# EUROPEAN PATENT SPECIFICATION

(11) **EP 2 550 036 B1**
(45) Date of publication and mention of the grant of the patent: **22.04.2020**
(21) Application number: 11758912.7
(22) Date of filing: 10.03.2011
(51) Int. Cl.: A61M 1/06

(54) **METHOD FOR DETERMINING FLOW RATES OF EXCRETED OR SECRETED BODY FLUIDS**
VERFAHREN ZUR BESTIMMUNG DER FLUSSRATEN AUSGESCHIEDENER ODER ABGESONDERTER KÖRPERFLÜSSIGKEITEN
PROCÉDÉ POUR DÉTERMINER LES DÉBITS DE FLUIDES CORPORELS EXCRÉTÉS OU SECRÉTÉS

(30) Priority: 25.03.2010 IL 20475210
(43) Date of publication of application: 30.01.2013
(73) Proprietor: Molex Ventures, LLC, Lisle, IL 60532 (US)
(72) Inventor: SELLA, Yoav, 58443 Holon (IL)
(74) Representative: Aronova
(86) International application number: PCT/IL2011/000232
(87) International publication number: WO 2011/117859

(56) References cited:
- EP-A1- 0 467 430
- WO-A1-89/11083
- WO-A1-2004/100788
- GB-A- 1 123 020
- GB-A- 2 173 905
- GB-A- 2 301 440
- US-A- 4 255 968
- US-A- 4 255 968
- US-A- 5 216 918
- US-A- 5 493 100
- US-A- 5 526 685
- US-A1- 2008 039 741
- US-A1- 2008 039 741
- US-A1- 2008 167 579

## Description

### FIELD OF THE INVENTION

The present invention relates to health care products and methods and more particularly to a method to determine and monitor the flow rates and volume of fluids excreted or secreted by the body.

### DESCRIPTION OF THE RELATED ART

Body fluids are liquids that are inside the bodies of humans and animals. They include fluids that are excreted or secreted from the body as well as body water that is normally retained inside the body. Excretion is the process of eliminating waste products of metabolism and other superfluous substances. It is an essential process in all forms of life. In contrast is secretion, where the fluid may have specific tasks after leaving the cell or organ.

An example of excretion can be found in the urinary system, where the urinary bladder is the excreting organ and the urine is the excreted fluid. An example of secretion can be found in lactation, where the mammary glands are the secreting organ and the fluid is milk ideally suited to babies.

The functioning of organs that perform the secretion and excretion of fluids can be diagnosed and studied by measuring the flow rate of the fluid. Current devices and methods for measuring flow rates of e.g., urine and breast fluid are based on weighing the accumulated fluid in a defined unit of time (1 minute, 24 hours, etc.). Urine flow rate is measured by uroflowmeters and lactation is measured by test weighing of babies.

These current procedures require rather sensitive and thus expensive scales. They also require the presence of the subject in a clinic - test-weighing, for instance, is done by weighing the subject (a baby) before a breastfeeding session as well as during and immediately after the session, for a period of 24 hours.

There are many fluid flow-meters on the market, but none are known which can be used to meet the exceptional challenges posed by lactation. A first difficulty lies in the direct transfer of the fluid from the breast of the woman nursing directly to the mouth of a baby, leaving no room for instrumentation. A second difficulty is the intermittent nature of the flow. Thirdly the actual flow rate is of little interest - the data required is usually the volume of milk drunk by a baby under special care.

A search of US patents and patent applications produced patents 5,571,226 to Palmer and 6,358,226 to Ryan which disclose electrically operated means for lactation, the milk being received in a container. Measuring a baby's consumption could be effected by pouring the extracted milk from these devices into the widely used graduated baby bottle, which allows convenient monitoring of the baby's fluid intake.

These devices require considerable power, the bulk of the apparatuses is cumbersome in mobile use, and the transfer of the milk from a first container to a second container is less hygienic than direct fluid passage from the woman to the baby. Other devices are known from WO 2004/100788 A1 and US 2008/0167579 A1.

### OBJECT OF THE INVENTION

An object of the present invention is therefore to solve the above discussed problems and to provide a method of measuring the rate and quantity of fluids secreted or excreted by the body, particularly milk being ingested from the breast by an infant.

It is a further object of the invention to provide a method which can be performed by non-medical persons in a private home without expert medical help.

### SUMMARY OF THE INVENTION

The present disclosure relates to an apparatus for determining the flow rate and amount of a selected secreted or excreted body fluid, comprising:
a) a measuring unit comprising a conduit made of a material having a low thermal conductivity and supporting at least two thermistors; an upstream thermistor serving as a compensation thermistor and a downstream thermistor located as far downstream as possible from said upstream thermistor and pre-heated to and kept at pre-defined temperature which is warmer than the fluid temperature to be metered;
b) means for applying small electric voltages to said thermistors to enable generation of an electric signal, said electric voltages flowing through said upstream thermistor being sufficiently low to prevent substantial heating of said thermistor; and
c) a control and display unit being operatively connected to said measuring unit, said control unit including means for calculating the difference between the electrical resistance of said upstream and said downstream thermistors and then calculating the flow rate based on the electrical resistance difference being a function of the body fluid flow rate and then calculating the accumulated amount of fluid based on the internal cross section of said conduit in which said fluid flows; and comprising a display for displaying the flow rate (optionally) and displaying the accumulating volume of body fluid being metered.

In a preferred embodiment of the present disclosure there is provided an apparatus further comprising a heating element, thermally coupled to said downstream thermistor and distant from said upstream compensation thermistor, said heating element heating said downstream thermistor and maintaining said downstream thermistor in a pre-defined temperature range.

An alternative embodiment of the apparatus further comprises a heating element, thermally coupled to said downstream thermistor and distant from said upstream compensation thermistor, said thermistors and heating element being supplied with a fixed constant regulated level of electric power.

In a further preferred embodiment of the present disclosure there is provided an apparatus wherein said heating element has a low electrical resistance and is powered by a low voltage power supply, the combination of which is sufficient to maintain said downstream thermistor at said constant pre-defined temperature.

In another preferred embodiment of the present disclosure there is provided an apparatus wherein said heating element is thermally coupled to said downstream thermistor by means of a heat conducting material.

In a further preferred embodiment of the present disclosure there is provided an apparatus wherein said connector means for interconnecting said control and display unit to said measuring unit comprises a connection selected from the group comprising electronic circuitry, a data and power communication cable, and an electromagnetic transmitter/receiver.

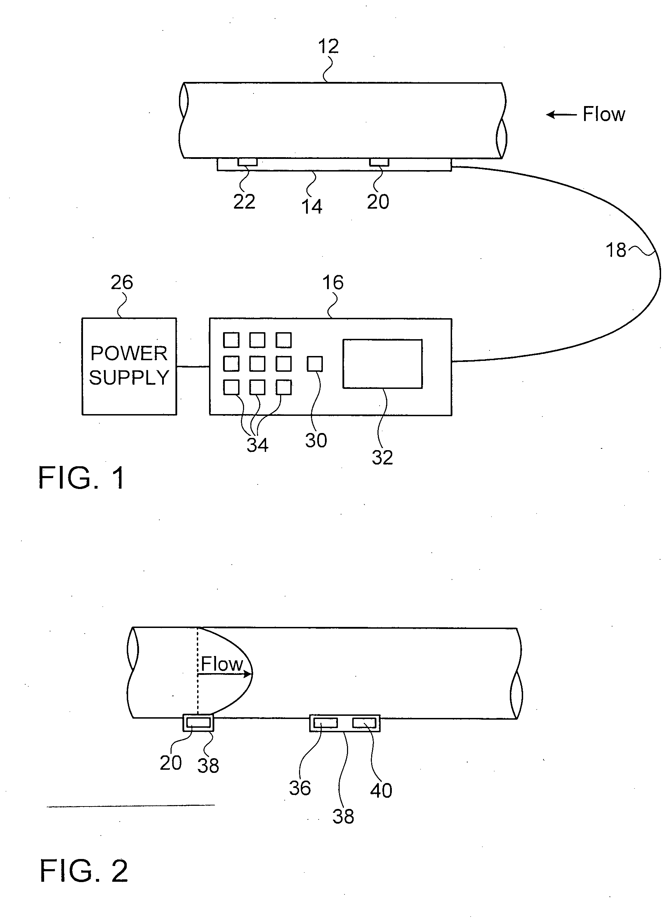

In a further preferred embodiment of the present disclosure there is provided an apparatus for determining the flow rate and amount of milk passing from the breast of a woman to a baby, comprising
a) a measuring unit having a short conduit containing at least two thermistors ;
b) an adaptor arranged to fit and retain contact with the nipple area of the breast, said adaptor being attached to an upstream extremity of said short conduit;
c) a baby nipple attached at a downstream extremity of said conduit;
   and
d) a control and display unit as seen in FIG.1 operatively connected to said measuring unit.

According to the present invention there is provided a method for determining the flow rate and amount of secreted or excreted body fluid, comprising the following steps:
step a) providing an apparatus according to an embodiment defined above; ;
step b) applying a voltage of about 15 - 50 volt across said downstream thermistor to serve as an integral heat source, the temperature of said downstream thermistor being held constant at about 39 - 45 degrees C;
step c) applying a low voltage of about 2 - 6 volts across said upstream thermistor to generate a electric measurement varying with the fluid flow with which said downstream thermistor is in direct thermal contact;
step d) preheating said apparatus;
step e) introducing the body fluid to be monitored into the upstream extremity of said conduit;
   and
step g) several times per second measuring the electric current flowing through both of said thermistors, and calculating the difference between said currents, and calculating the fluid flow rate as a function of thermistor resistance while using a memory component to effect corrections as required for any particular unit, and integrating the series of values over time to arrive at the increasing volume of fluid having passed through said conduit and displaying the value of said volume.

In a most preferred embodiment of the present invention there is provided a method for determining the flow rate and amount of secreted or excreted body fluid except that in step c) said voltage applied across said downstream thermistor is less than 15 volts and heating of said downstream thermistor is effected by a resistance element in close thermal contact with said downstream thermistor and with said body fluid.

The use of thermistors ensures high sensitivity through large resistance change (negative change - for NTC thermistor, or positive change - for PTC thermistors) and corresponding large voltage signal for small changes in their temperature, induced by the fluid, at no-flow situation or during constant or varying flow rates. As such, the use of thermistors is preferable, but other electrical resistance elements, presenting resistance change over temperature, can also be used.

The apparatus includes a measuring unit and a control and display unit. Depending on the required configuration of the apparatus, a connector cable, an electronic circuitry, a data communication cable, or electromagnetic transmitter/receiver, may be used for connecting the measuring unit to the control and display unit.

The measuring unit may comprise a set of two or three electrical components - two thermistors (NTC or PTC) or a resistor and two thermistors (NTC or PTC), mounted, in direct contact with the fluid, within the wall of a conduit that defines an outlet through which the fluid passes.

The display means may comprise a liquid crystal or similar display, and/or may be adapted to print a graphical representation of the data received from measuring unit. The display means may also include a number of keys for entering data (e.g. the name of the subject, his age, etc.) or for selectively display of the flow rate or amount of fluid per measuring session, the accumulating amount of fluid in several measuring sessions, and previously measured fluid flow rates or accumulated amounts.
Thus, the apparatus according to the invention provides the subject, in real time, with an indication of the volume of fluid that is excreted or secreted from the body. It would accordingly no longer be necessary to follow the cumbersome weighing process mentioned hereinbefore.

The invention will now be described further with reference to the accompanying drawings, which represent by example preferred embodiments of the invention. Structural details are shown only as far as necessary for a fundamental understanding thereof. The described examples, together with the drawings, will make apparent to those skilled in the art how further forms of the invention may be realized.

### BRIEF DESCRIPTION OF THE DRAWINGS

In the drawings:
FIG. 1 is a diagrammatic view of a preferred embodiment of the apparatus according to the disclosure;
FIG. 2 is an enlarged sectional view of a part of the apparatus having an auxiliary heating element;
FIG. 3 is a schematic view of an embodiment wherein the measuring unit is connected to the control and display unit by a radio or infrared link;
FIG. 4 is a sectional view of an apparatus arranged for monitoring lactation;
FIG. 5 is a diagram of a method which operates without any auxiliary heater;
   and
FIG. 6 is a diagram of a method which operates with an auxiliary heater at low voltage.

### DETAILED DESCRIPTION OF THE INVENTION

There is seen in FIG. 1 an apparatus 10 for determining the flow rate and amount of a selected secreted or excreted body fluid passing through a conduit 12.
The apparatus 10 comprises a measuring unit 14 , a control and display unit 16, a data and power link 18 interconnecting these units, and a power supply 26, which can be integral to the control and display unit 16.
The measuring unit 14 comprises a conduit 12 made of a material having a low thermal conductivity and supporting two thermistors 20, 22. The conduit 12 is made of a grade suitable for the transmission of the measured fluid, e.g., food grade material for lactation measurement. The low thermal conductivity of the conduit material allows for reducing the heat loss from the heated thermistor 22 to the environment and thus allows effective heat transfer from the electrical components 20, 22, directly to the measured fluid.
The upstream thermistor 20 serves as a compensation thermistor. The electrically heated downstream thermistor 22 is wired in parallel to the upstream thermistor. The thermistors 20, 22 are located as far away as possible from each other. The downstream thermistor 22 is cooled by the body fluid flowing through the conduit 12, and is held at pre-defined temperature which is about 2 - 8 degrees warmer than the temperature of the body fluid being metered. Preferably, both thermistors 20, 22 are surface mounted devices mounted on a flexible printed circuit board 24 seen in FIG 2. Both thermistors 20, 22 are mounted in direct contact with the fluid within the conduit 12 through which the body fluid passes.
With regard to applying electric voltages to the thermistors 20, 22 to enable the generation of an electric signal, in the present embodiment the needed power can be supplied by a multi-voltage transformer/rectifier 26 or a similar power supply which is arranged to provide over 15 volt to the downstream thermistor. The precise voltage is arranged to be sufficient to heat the thermistor between 2 and 8 degrees C above the temperature of the body fluid being metered. Thus there is no requirement for an auxiliary heater.

The control and display unit 16 is operatively connected to the measuring unit 14 in the present embodiment by means of a flexible cable 18.
The control and display unit 16 includes means for calculating the difference between the electrical signals from the upstream and the downstream thermistors 20, 22 and then calculating the fluid flow rate based on these values. The difference between the two values is a function of the body fluid flow rate. The control unit 16 then calculates the accumulated amount of fluid which has passed through the conduit by an integration function based on the internal cross section of the conduit 12 in which the fluid flows.
The electrical signal of the upstream compensation thermistor 20 will relate to the temperature of the fluid. This value is deducted from the electrical signal value registered by the downstream measuring thermistor 22. The compensated electrical signal value registered by the downstream measuring thermistor is an independent signal that is free of the influence of temperature changes of the fluid. Keys 34 are provided to enable users to enter data such as the name of the baby, date and time etc. These calculations are easily performed by a microprocessor 30 located in the control and display unit 16. Readings are taken and processed many times per second. The microprocessor 30 uses these readings and the calibration data in the memory component of the measuring unit to convert the signal to flow data. The flow data is then integrated over time to give the accumulating amount of fluid which has passed through the conduit, based on the cross section area thereof. This value is then shown on the LCD display 32. Optionally the display 32 also shows the flow rate.
In the present higher voltage apparatus, the temperature rise is induced by applying a voltage directly to the thermistor 22. The thermistor 32 is encapsulated using a highly thermally conductive compound 38, seen in FIG. 2, for electrically isolating the thermistor from the fluid, while being in direct contact with it.

With reference to the rest of the figures, similar reference numerals have been used to identify similar parts.

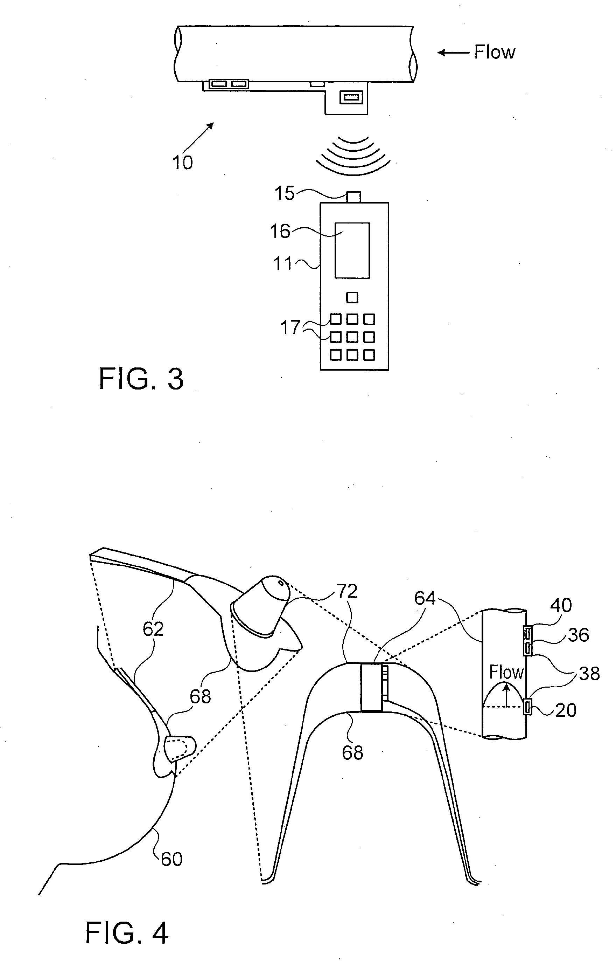

Referring now to FIG. 2, there is seen an apparatus 38 further comprising a heating element 36, which is thermally coupled to the downstream thermistor 22 and distant from the upstream compensation thermistor 20 seen in FIG. 1. Using a separate resistor 36 for heating purposes, allows use of a high resistance thermistor 40 for flow measurement. The use of a separate heating resistor also eliminates the self heating effects of the thermistor 40, and allows a low current electrical circuit to be used to generate the electric signal from the thermistor 40.
The heating element 36 raises the temperature of the downstream thermistor 40, and maintains the downstream thermistor in a pre-defined temperature range. The precise voltage is arranged to be sufficient to heat the thermistor between 2 and 8 degrees C above the temperature of the body fluid being metered.
Alternatively the downstream thermistor 40 is provided with a controlled quantity of power, and fluid flow is calculated from the temperature loss of the thermistor 40.
As will be seen in FIG. 4, with regard to applying small electric voltages to the thermistors 20, seen in FIG 1, and the thermistor 40 to enable the generation of an electric signal, in the present embodiment the needed power can be supplied by batteries 42 which are small enough to be housed in the control and display unit 44. It should be noted that the electrical signal generated by the thermistor 20 is required to be sufficiently low to prevent substantial heating thereof, while maintaining the downstream thermistor at the constant pre-defined temperature.
Preferably the heating element 36 is thermally coupled to the downstream thermistor 40 by means of a heat conducting material, for example copper.
When there is a flow of a body fluid the electric signal value received from the upstream compensation thermistor 20 will register the value that corresponds to the temperature of the body fluid, while the electric signal obtained from the downstream measuring thermistor 40 will change as a function of the flow rate - the higher the flow rate the larger the change. The heating element 36 provides heat to this thermistor 40, to return same to its original value after flow ceases.
The electrical signal values of the thermistors 20, 40 when in use, which in turn represent thermistor temperature, and the reduction of temperature on the downstream thermistor 40 indicates the flow rate of the body fluid which acts as a coolant.

FIG. 3 illustrates an apparatus 46 wherein the means for interconnecting the control and display unit 48 to the measuring unit 50 comprises an electromagnetic transmitter/receiver 52, 54.
Electric power is preferably supplied by batteries 42 in the measuring unit 50, while either batteries or a transformer/rectifier supply power 26 is contained in the control and display unit 48.

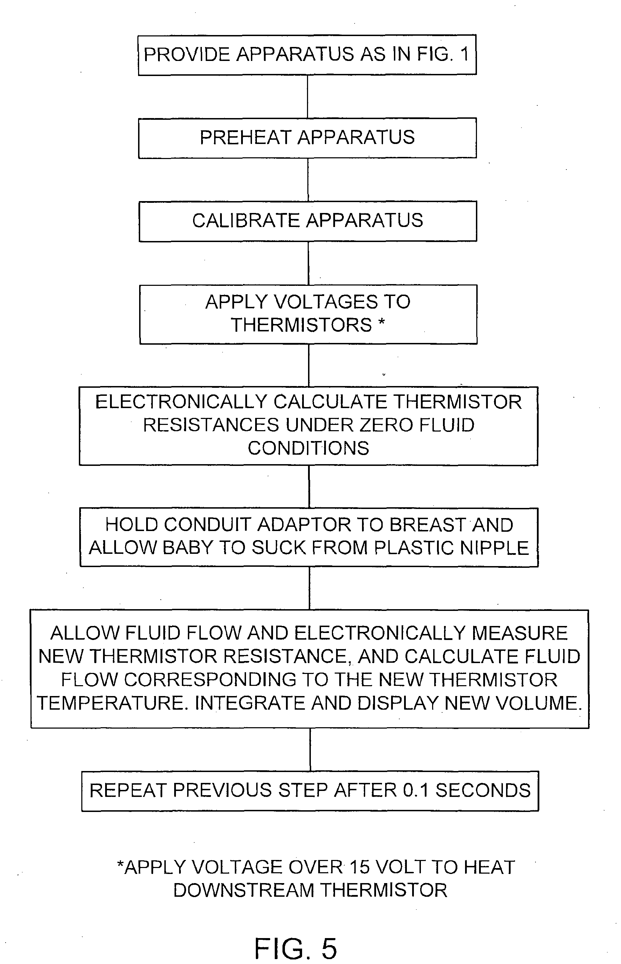

Seen in FIG. 4 is an apparatus 58 adapted for the specific purpose of determining the flow rate and amount of milk passing from the breast 60 of a woman to a baby (not shown).
A measuring unit 62 has a short conduit 64 containing two thermistors 20, 22 as described with reference to FIG. 1. This unit 62 is held in position by a vest, a bra or a special belt 66 which is arranged to support the measuring unit 62.
A flexible adaptor 68 is arranged to fit and retain contact with the nipple area of the breast 60, the adaptor 68 being attached to the upstream extremity 70 of the short conduit 64.
An elastomer baby nipple 72 is attached at a downstream extremity 74 of the conduit 64.
The adaptor 68 and nipple 72 can be molded as an integrated unit.
A control and display unit 16 as described with reference to FIG. 1 is operatively connected to the measuring unit 62 by a cable 18.
Referring now to FIG. 5, there is depicted diagrammatically a method for determining the flow rate and amount of secreted or excreted body fluid, comprising the following steps:
STEP a) providing an apparatus as seen in FIG.1.
   As minor variations occur during the production process of the measuring unit, and since each electronic component in the measuring unit is inherently different, a calibration of this unit is required. A memory component (e.g., EPROM) may be used to store calibration data. The calibration data should represent the true resistance-temperature data of the components in the measuring unit.
STEP b) applying a voltage 82 of about 15 - 50 volt across the downstream thermistor to serve as an integral heat source, 82 the temperature of the downstream thermistor being held constant at about 39 - 45 degrees C, or the thermistor being supplied with a precisely constant regulated power level;
   Using the available calibration data, stored in the memory component of the measuring unit the electrical signal generated on the thermistors can then be corrected and used for calculating the flow rate. This is done by the microprocessor (or microcontroller) inside the control and display unit in which a pre-defined table (or equation) is stored, representing the specific electrical signal that corresponds to the specific flow rate.
STEP c) applying a voltage 82 of about 2 - 6 volts across the upstream thermistor to generate an electric signal varying with the fluid flow with which the downstream thermistor is in direct thermal contact;
STEP d) preheating 78 of the apparatus;
   The pre-heating is essential for eliminating transient phenomena of resistance change of the thermistor when the flow starts and it allows for measurement to start immediately at the start of the flow. The apparatus can thus continuously measure the fluid flow (usually non stable, or oscillating flow), from start, based on small changes from a baseline resistance of the thermistor, corresponding to the pre-defined temperature. These small changes correspond to the fluid flow, and allow fast response of the measuring unit.
   The correct pre-heating of the resistor, or the thermistor, should be determined based on the fluid to be measured (for e.g. retaining the original characteristics of the fluid, maintaining the overall original temperature of the fluid, etc.) and based on the thermal behavior of the thermistor e.g., the resistance-temperature behavior at the relevant temperature ranges, the maximum allowed power over the component, etc.. For example, the initial temperature of the thermistor in a lactation measurement apparatus may be predefined to be between 39-45 °C, based on the fact that the breast milk exits the body at 35-37 °C at the skin surface, and based on need to maintain the overall temperature of the fluid in human-relevant temperatures.
STEP e) introducing the body fluid 86 to be monitored into the upstream 88 extremity of the conduit;
   and
STEP g) 10 - 200 times per second measuring 90 the voltage over both of the thermistors, and calculating the difference between the voltages, and calculating 84 the fluid flow rate as a function of the downstream thermistor's compensated voltage while using a memory component to effect corrections as required for any particular unit, and integrating the series of values over time to arrive at the increasing volume of fluid having passed through the conduit and displaying the value of the volume.

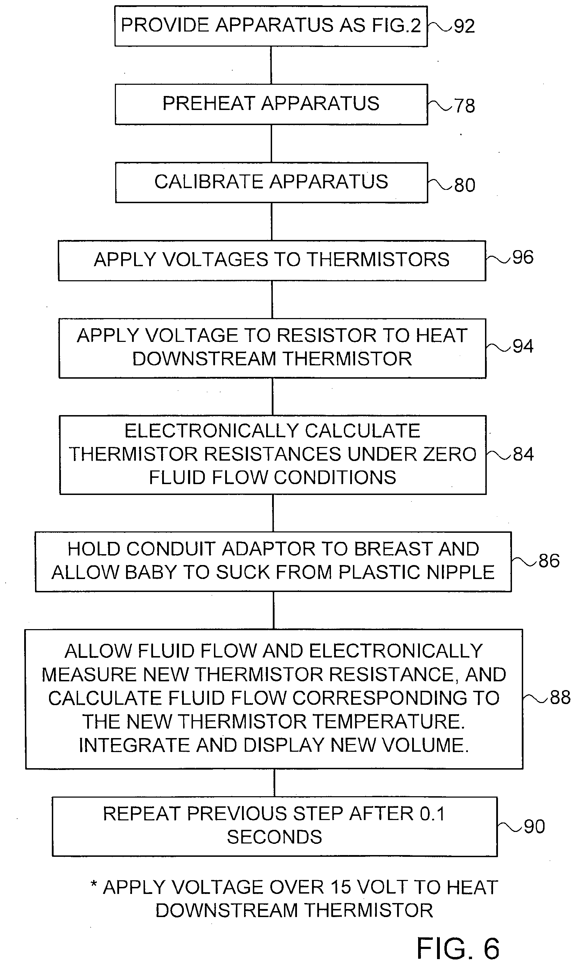

FIG. 6 represents a second method for determining the flow rate and amount of secreted or excreted body fluid. The second method 78 - 96 is similar to that described with reference to FIG. 5 except that the downstream thermistor is heated 94 by an electric resistance element. The advantage of this arrangement is in obviating the need to apply a higher voltage to the downstream thermistor.
Accordingly in STEP c) the voltage applied 96 across the downstream thermistor is less than 15 volts and heating of the downstream thermistor is effected by said resistance element in close thermal contact with the downstream thermistor and with the body fluid.
The temperature of the thermistor should preferably be raised, by pre-heating of the heating element (resistor or thermistor itself) to several degrees higher than the expected temperature of the fluid, prior to the measurement starting point. Either the downstream thermistor is kept at this pre-defined constant temperature, by starting and stopping the power supply to the heating element based on the resistance value of the thermistor that corresponds to its temperature, or this thermistor is supplied with a precisely constant power level..
Using the available calibration data, stored in the memory component of the measuring unit the electrical signal generated on the thermistors is then corrected and used for calculating the flow rate. This is done by the microprocessor (or microcontroller) inside the control and display unit in which a pre-defined table (or equation) is stored, representing the specific voltage signal that corresponds to the specific flow rate.

The scope of the described invention is intended to include all embodiments coming within the meaning of the following claims. The foregoing examples illustrate useful forms of the invention, but are not to be considered as limiting its scope, as those skilled in the art will be aware that additional variants and modifications of the invention can readily be formulated without departing from the meaning of the following claims.

## Claims

1. A method for determining the flow rate and amount of secreted or excreted body fluid, comprising the following steps:
step a) providing an apparatus for determining the flow rate and amount of a selected secreted or excreted body fluid, comprising:
∘ a measuring unit (14) comprising a conduit (12) made of a material having a low thermal conductivity and supporting at least an upstream thermistor (20) serving as a compensation thermistor and a downstream thermistor (22, 40) located as far downstream as possible from said upstream thermistor;
∘ means (26) for applying a constant small electric voltages to said thermistors to enable generation of an electric signal; and
∘ a control and display unit (16) being operatively connected to said measuring unit, said control unit including means (30) for calculating the difference between the electrical resistance of said upstream and said downstream thermistors and then calculating the flow rate based on the electrical signal difference being a function of the body fluid flow rate and then calculating the accumulated amount of fluid based on the internal cross section of said conduit in which said fluid flows; and comprising a display (32) for displaying the accumulating volume of body fluid being metered and optionally displaying the flow rate;
step b) applying a voltage of about 15 - 50 volt across said downstream thermistor to serve as an integral heat source such that the temperature of said downstream thermistor being held constant at about 39 - 45°C;
step c) applying a voltage of about 2 - 6 volts across said upstream thermistor to generate an electric signal sufficiently low to prevent substantial heating of said thermistor and varying with the fluid flow with which said downstream thermistor is in direct thermal contact;
step d) preheating of said apparatus, the preheating comprising preheating the downstream thermistor to a temperature which is warmer than the fluid temperature to be metered;
step e) introducing the body fluid to be monitored into the upstream extremity of said conduit; and
step g) 10 - 200 times per second measuring the voltage over both of said thermistors, and calculating the difference between these electric signals, and calculating the fluid flow rate as a function of the calculated difference while using a memory component to effect corrections as required for any particular unit, and optionally integrating the series of values over time to arrive at the increasing volume of fluid having passed through said conduit and displaying the value of said volume.

2. The method for determining the flow rate and amount of secreted or excreted body fluid as claimed in claim 1, except that in step b) said voltage applied across said downstream thermistor is less than 15 volts and heating of said downstream thermistor is effected by a resistance element (36) of said apparatus, said resistance element being arranged in close thermal contact with said downstream thermistor.

3. The method according to claim 2, comprising heating said downstream thermistor and maintaining said downstream thermistor in a predefined temperature range by said resistance element (36), said element being thermally coupled to said downstream thermistor and distant from said upstream compensation thermistor.

4. The method according to of claim 3, wherein said resistance element has a low electrical resistance and is powered by a low voltage power supply, the combination of which is sufficient to maintain said downstream thermistor at said constant pre-defined temperature.

5. The method according to claim 3, wherein said resistance element is thermally coupled to said downstream thermistor by means of a heat conducting material.

6. The method according to claim 2, further comprising supplying said thermistors and resistance element with a fixed constant regulated level of electric power, the resistance element being thermally coupled to said downstream thermistor and distant from said upstream compensation thermistor.

7. The method according to any one of claims 1 to 6 for determining the flow rate and amount of milk passing from the breast of a woman to a baby, the method comprising arranging an adaptor (68) to contact a nipple area of the breast, the adaptor being arranged to fit and retain the contact, the adaptor being attached to an upstream extremity of said conduit, and attaching a baby nipple at a downstream extremity of said conduit.

## Patentansprüche

1. Verfahren zur Bestimmung der Flussrate und der Menge einer ausgeschiedenen oder abgesonderten Körperflüssigkeit, umfassend die folgenden Schritte:
Schritt a) Bereitstellen einer Vorrichtung zur Bestimmung der Flussrate und der Menge einer ausgeschiedenen oder abgesonderten Körperflüssigkeit, umfassend:
o eine Messeinheit (14), umfassend eine Leitung (12) aus einem Material mit einer geringen Wärmeleitfähigkeit, die zumindest einen stromaufwärtigen Thermistor (20), der als ein Ausgleichsthermistor dient, und einen stromabwärtigen Thermistor (22, 40), der sich soweit als möglich stromabwärts von dem stromaufwärtigen Thermistor befindet, trägt;
o Mittel (26) zum Anlegen einer konstanten kleinen elektrischen Spannung an die Thermistoren, um die Erzeugung eines elektrischen Signals zu ermöglichen; und
o eine Steuer- und Anzeigeeinheit (16), die betriebsmäßig mit der Messeinheit verbunden ist, wobei die Steuereinheit Mittel (30) zum Berechnen der Differenz zwischen dem elektrischen Widerstand der stromaufwärts und stromabwärts gelegenen Thermistoren und dann Berechnen der Flussrate basierend auf der elektrischen Signaldifferenz, die eine Funktion der Flussrate der Körperflüssigkeit ist, und dann Berechnen der akkumulierten Menge der Flüssigkeit basierend auf dem inneren Querschnitt der Leitung, in der die Flüssigkeit fließt, einschließt; und eine Anzeige (32) umfassend zum Anzeigen des akkumulierenden Volumens der zu messenden Körperflüssigkeit und um optional die Flussrate anzuzeigen;
Schritt b) Anlegen einer Spannung von etwa 15-50 Volt über dem stromabwärtigen Thermistor, um als eine integrale Wärmequelle zu dienen, auf derartige Weise, dass die Temperatur des stromabwärtigen Thermistors konstant bei etwa 39-45 °C gehalten wird;
Schritt c) Anlegen einer Spannung von etwa 2-6 Volt über dem stromaufwärtigen Thermistor, um ein elektrisches Signal zu erzeugen, das ausreichend niedrig ist, um ein wesentliches Erwärmen des Thermistors zu verhindern, und das mit dem Flüssigkeitsstrom, mit dem der stromabwärtige Thermistor in direktem Wärmekontakt steht, variiert;
Schritt d) Vorwärmen der Vorrichtung, wobei das Vorwärmen das Vorwärmen des stromabwärtigen Thermistors auf eine Temperatur umfasst, die wärmer ist als die zu messende Flüssigkeitstemperatur;
Schritt e) Einbringen der zu überwachenden Körperflüssigkeit in das stromaufwärtige Ende der Leitung; und
Schritt g) 10-200 mal pro Sekunde Messen der Spannung über beiden Thermistoren und Berechnen der Differenz zwischen diesen elektrischen Signalen und Berechnen der Flussrate der Flüssigkeit als eine Funktion der berechneten Differenz, während eine Speicherkomponente verwendet wird, um Korrekturen zu bewirken, wenn eine solche für irgendeine bestimmte Einheit erforderlich ist, und wahlweise Integrieren der Reihe von Werten im Laufe der Zeit, um das zunehmende Volumen der Flüssigkeit zu erreichen, die durch die Leitung gelaufen ist, und Anzeigen des Wertes des Volumens.

2. Verfahren zum Bestimmen der Flussrate und der Menge einer ausgeschiedenen oder abgesonderten Körperflüssigkeit nach Anspruch 1, mit der Ausnahme, dass in Schritt b) die an den stromabwärtigen Thermistor angelegte Spannung weniger als 15 Volt beträgt und das Erwärmen des stromabwärtigen Thermistors durch ein Widerstandselement (36) der Vorrichtung bewirkt wird, wobei das Widerstandselement in engem Kontakt mit dem stromabwärtigen Thermistor angeordnet ist.

3. Verfahren nach Anspruch 2, umfassend das Erwärmen des stromabwärtigen Thermistors und das Halten des stromabwärtigen Thermistors in einem vordefinierten Temperaturbereich durch das Widerstandselement (36), wobei das Element mit dem stromabwärtigen Thermistor wärmegekoppelt und entfernt von dem stromaufwärtigen Kompensationsthermistor ist.

4. Verfahren nach Anspruch 3, wobei das Widerstandselement einen geringen elektrischen Widerstand aufweist und durch eine Niederspannungsversorgung gespeist wird, wobei diese Kombination ausreichend ist, um den stromabwärtigen Thermistor bei der konstanten vordefinierten Temperatur zu halten.

5. Verfahren nach Anspruch 3, wobei das Widerstandselement mit dem stromabwärtigen Thermistor mittels eines Wärmeleitmaterials wärmegekoppelt ist.

6. Verfahren nach Anspruch 2, ferner umfassend das Versorgen der Thermistoren und des Widerstandselements mit einem festen konstanten regulierten Niveau elektrischer Leistung, wobei das Widerstandselement mit dem stromabwärtigen Thermistor wärmegekoppelt und entfernt von dem stromaufwärtigen Kompensationsthermistor ist.

7. Verfahren nach einem der Ansprüche 1 bis 6 zur Bestimmung der Flussrate und der Menge an Milch, die von der Brust einer Frau zu einem Baby strömt, wobei das Verfahren das Anordnen eines Adapters (68) in Kontakt mit einem Brustwarzenbereich der Brust umfasst, wobei der Adapter angeordnet ist, um zu passen und den Kontakt zu halten, wobei der Adapter an ein stromaufwärtiges Ende der Leitung angebracht ist, und Anbringen eines Babysaugers an ein stromabwärtiges Ende der Leitung.

## Revendications

1. Procédé de détermination du débit et de la quantité de fluide corporel sécrété ou excrété, comprenant les étapes suivantes :
étape a) fourniture d'un appareil pour déterminer le débit et la quantité d'un fluide corporel sécrété ou excrété sélectionné, comprenant :
o une unité de mesure (14) comprenant un conduit (12) constitué d'un matériau ayant une faible conductivité thermique et supportant au moins une thermistance amont (20) servant de thermistance de compensation et une thermistance aval (22, 40) située aussi loin que possible en aval de ladite thermistance amont ;
o un moyen (26) pour appliquer de petites tensions électriques constantes auxdites thermistances pour permettre la génération d'un signal électrique ; et
o une unité de commande et d'affichage (16) étant opérationnellement connectée à ladite unité de mesure, ladite unité de commande incluant un moyen (30) pour calculer la différence entre la résistance électrique desdites thermistances amont et aval puis calculer le débit sur la base de la différence de signal électrique étant une fonction du débit de fluide corporel puis calculer la quantité cumulée de fluide sur la base de la coupe transversale interne dudit conduit dans lequel ledit fluide s'écoule ; et comprenant un affichage (32) pour afficher le volume cumulé de fluide corporel étant dosé et éventuellement afficher le débit ;
étape b) application d'une tension d'environ 15 à 50 volts à travers ladite thermistance aval pour servir de source de chaleur intégrée de telle sorte que la température de ladite thermistance aval est maintenue constante à environ 39 à 45 °C ;
étape c) application d'une tension d'environ 2 à 6 volts à travers ladite thermistance amont pour générer un signal électrique suffisamment bas pour empêcher un chauffage sensible de ladite thermistance et faire varier l'écoulement de fluide avec lequel ladite thermistance aval est en contact thermique direct ;
étape d) préchauffage dudit appareil, le préchauffage comprenant le préchauffage de la thermistance aval à une température qui est plus chaude que la température de fluide à doser ;
étape e) introduction du fluide corporel à surveiller dans l'extrémité amont dudit conduit ; et
étape g) 10 à 200 fois par seconde, mesure de la tension sur l'une et l'autre desdites thermistances, et calcul de la différence entre ces signaux électriques, et calcul du débit de fluide en fonction de la différence calculée tout en utilisant un composant de mémoire pour réaliser des corrections selon le besoin pour n'importe quelle unité particulière, et éventuellement intégration de la série de valeurs au fil du temps pour arriver au volume croissant de fluide étant passé à travers ledit conduit et affichage de la valeur dudit volume.

2. Procédé de détermination du débit et de la quantité de fluide corporel sécrété ou excrété selon la revendication 1, si ce n'est qu'à l'étape b) ladite tension appliquée à travers ladite thermistance aval est inférieure à 15 volts et le chauffage de ladite thermistance aval est effectué par un élément de résistance (36) dudit appareil, ledit élément de résistance étant agencé en contact thermique rapproché avec ladite thermistance aval.

3. Procédé selon la revendication 2, comprenant le chauffage de ladite thermistance aval et le maintien de ladite thermistance aval dans une plage de température prédéfinie par ledit élément de résistance (36), ledit élément étant thermiquement couplé à ladite thermistance aval et distant de ladite thermistance de compensation amont.

4. Procédé selon la revendication 3, dans lequel ledit élément de résistance a une faible résistance électrique et est alimenté par une alimentation électrique basse tension, dont la combinaison est suffisante pour maintenir ladite thermistance aval à ladite température prédéfinie constante.

5. Procédé selon la revendication 3, dans lequel ledit élément de résistance est couplé thermiquement à ladite thermistance aval au moyen d'un matériau thermoconducteur.

6. Procédé selon la revendication 2, comprenant en outre l'alimentation desdits thermistances et élément de résistance avec un niveau régulé constant fixe de puissance électrique, l'élément de résistance étant couplé thermiquement à ladite thermistance aval et distant de ladite thermistance de compensation amont.

7. Procédé selon l'une quelconque des revendications 1 à 6 pour déterminer le débit et la quantité de lait passant du sein d'une femme à un bébé, le procédé comprenant la mise en place d'un adaptateur (68) pour venir en contact avec une zone de mamelon du sein, l'adaptateur étant agencé pour ajuster et maintenir le contact, l'adaptateur étant fixé à une extrémité amont dudit conduit, et la fixation d'une tétine au niveau d'une extrémité aval dudit conduit.
